(19) Europäisches Patentamt
European Patent Office
Office européen des brevets

(11) Publication number: **0 184 861**
**B1**

(12) **EUROPEAN PATENT SPECIFICATION**

(45) Date of publication of patent specification: **06.06.90**

(21) Application number: **85200443.1**

(22) Date of filing: **25.03.85**

(51) Int. Cl.$^5$: **C 07 D 207/273**, C 07 D 409/12, C 07 D 405/12, C 07 D 417/12, A 61 K 31/40

(54) **Derivatives of the 3-mercapto-2-oxo-1-pyrrolidinacetic acid, process for their preparation and pharmaceutical compositions containing them.**

(30) Priority: **30.03.84 IT 2031584**

(43) Date of publication of application: **18.06.86 Bulletin 86/25**

(45) Publication of the grant of the patent: **06.06.90 Bulletin 90/23**

(84) Designated Contracting States: **AT BE CH DE FR GB LI LU NL SE**

(56) References cited:
**EP-A-0 071 216**
**US-A-4 138 495**

(73) Proprietor: **PROTER S.p.A.**
**38 Via Lambro**
**I-20090 Opera (Milan) (IT)**

(72) Inventor: **Dall'Asta, Leone**
**Viale Damiano Chiesa 26**
**I-27100 Pavia Milano (IT)**
Inventor: **Coppi, Germano**
**Via Morandi**
**I-20094 Buccinasco Milano (IT)**
Inventor: **Scevola, Mario Ercole**
**Via Tuberose 4**
**I-20146 Milano (IT)**

(74) Representative: **Gervasi, Gemma, Dr.**
**NOTARBARTOLO & GERVASI Srl 33,**
**Viale Bianca Maria**
**I-21100 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 184 861 B1**

**Description**

The present invention relates to one of its features, to compounds having the formula I:

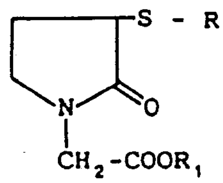

(I),

wherein R is hydrogen, $-COR_2$, $-CH_2COOR_3$, in which $R_2$ is a $C_2-C_8$ linear or branched chain alkyl, or a phenyl, a heterocyclic radical containing 1, 2 or 3 hetero atoms from among sulphur, nitrogen, oxygen, such as 2-thienyl, 2-furyl, 4-thiazolyl, 1,3,4-thiadiazol-2-yl groups and in which $R_3$ is hydrogen, a linear or branched chain $C_1-C_4$ alkyl and $R_2$ is hydrogen or a linear or branched chain $C_1-C_8$ alkyl and to the pharmaceutically acceptable salts of the compounds of formula I wherein one or both the substituents R and $R_1$ or $R_3$ and $R_1$ are hydrogen; in fact, when R and/or $R_1$ and/or $R_3$ are hydrogen, the —SH and —COOH groups can be salified with an inorganic or organic pharmaceutically acceptable base. Preferred salts are those with alkyl metals, such as sodium and potassium, with earth-alkaly metals such as magnesium and calcium, with organic bases, such as diethanolamine, tromethamine or with amphoteric products such as lysine or arginine.

According to another feature, the present invention relates to a process for the preparation of the compounds of formula I and, possibly, of their salts with inorganic or organic bases, characterized in that a bromopyrrolidin-2-one derivative of formula II:

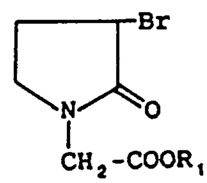

(II),

wherein $R_1$ has the above definite meaning, is treated with a thiolic compound of formula III:

$$HS-R \qquad (III)$$

wherein R has the above definite meaning, or with an alkali salt thereof, possibly in the presence of an inorganic or organic base in an organic solvent at a temperature between 0°C and +40°C and the thus obtained product is then possibly converted into its pharmaceutically acceptable salts.

The starting compounds of formula II are known in the literature and may be prepared by reacting 2,4-dibromo-butiryl bromide with glycine ethylester and subsequent cyclization of the N-(2,4-dibromobutiryl)glycine ethylester with sodium methoxide with methanol (Il Farmaco, Ed. sci. 1978, 33, 130—141).

The thiolic compounds of formula III are well known and may be used in acidic form or as one of their alkali salts, preferably sodium or potassium salts. In the case of the preparation of the compounds of formula I wherein R is hydrogen, it is suitable to use an alkali hydrosulfide, preferably NaSH or KSH. When the reaction is carried out in the presence of an organic or inorganic base, the latter may be an alkali hydroxide or carbonate, or an organic base such as triethylamine.

The reaction is as a rule completed after 2—8 hours at a temperature of between 0°C and +40°C and the final product is isolated according to the standard methods and possibly salified by treatment with an inorganic or organic base.

The process for the preparation of the compounds of formula I is a wholly general method. However the possible simultaneous presence of an acid and of an ester, of a free sulfidryl group or of thioesters, leads to variations of the general method which may result in increased yields.

For instance, in the case of the preparation of compounds of formula I in which R is hydrogen, it may be suitable to have recourse in a first time to the preparation of the corresponding S-benzoyl derivative (formula I, $R=CO-C_6H_5$) and to the subsequent removal of the benzoyl group.

It may also be convenient to have recourse to the S-benzoyl derivative, which is obtained with high yields from the starting compounds of the formula II, in the preparation of less reactive thioesters, which can be readily obtained, after removal of the benzoyl group, by reaction with the chloride of the suitable acid.

The above variations are illustrated by the scheme of preparation of the compounds of formula IV wherein $R_1=H$ and $R=CH_2-COOH$, of formula V wherein $R=R_1=H$ and of formula VI wherein $R_1=H$ and R=(2-thienyl)carbonyl.

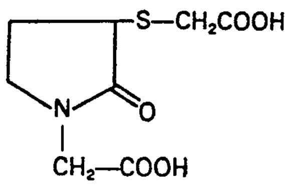

(IV)

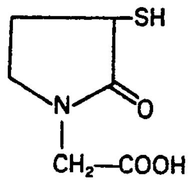

(V)

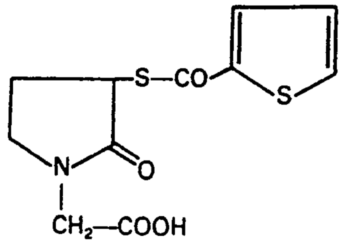

(VI)

according to the following preferred method:

a) hydrolysis of the ethyl ester of 3-bromo-2-oxo-pyrrolidin-1-yl acetic acid (IIa) in alcohol solution in the presence of a calculated amount of N NaOH water solution and subsequent acidification to give the corresponding acid (IIb).

b) reaction of the acid IIb with potassium thiobenzoate in aqueous solution to give 3-thiobenzoyl-2-oxo-pyrrolidin-1-yl acetic acid.

c) removal of the benzoyl group in aqueous solution of ammonium hydroxide to give the compound V.

d) esterification of V, as sodium salt, with the chloride of 2-thiophen-carboxylic acid to give the compound VI.

e) reaction of IIb with thioglycolic acid $HS—CH_2COOH$, to give the compound IV.

The hydrolysis a) is preferably carried out in a reaction medium from the group formed by water, ethanol, methylcellosolve, dimethylsulfoxide,

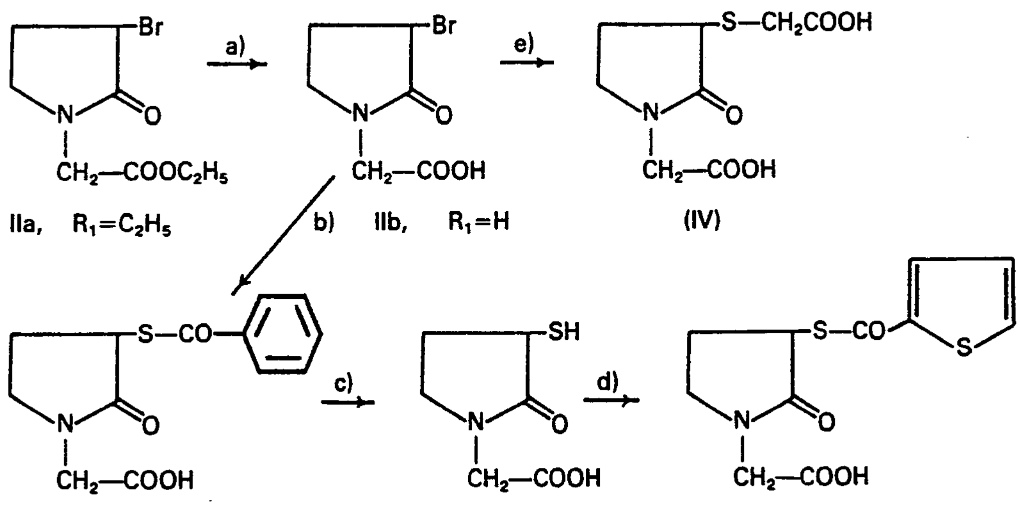


IIa, $R_1=C_2H_5$ IIb, $R_1=H$ (IV)

(VII) (V) (VI)

ethylene glycol, dioxane or their mixtures.

The suitable temperature is of between 0°C and +50°C; the reaction time varies with the temperature. At +20°C the hydrolysis reaction in aqueous ethanol is completed after 4—5 hours.

The isolation of the product IIb takes place by neutralization with diluted HCl, subsequent concentration and extraction of the residue with a suitable solvent, for instance ethyl acetate.

From the organic phase the product is isolated by evaporation under reduced pressure. The reaction b) to give VII is carried out in water solution wherein the compound IIb, as the sodium salt, is contacted with potassium thiobenzoate. At temperatures of between +10° and +50°C, preferably at +20°/+25°C a time of 5 hours is required for the completion of the reaction. After acidification the product is extracted with a suitable solvent, for instance ethyl acetate. From the organic phase the product is isolated by concentration under vacuum.

The removal of the benzoyl group (reaction c) to give the compound V is carried out in aqueous environment by addition of an alkali hydrate, preferably ammonium hydroxide.

At temperatures of between +10°C and +50°C, preferably at +15°C/+20°C the reaction is completed after 5 hours.

After removal of the benzamide by filtration, the aqueous solution is evaporated under vacuum at +50°C and the residue, after acidification is extracted with a suitable solvent, for instance ethyl acetate, and subsequently the product V is isolated by concentration under reduced pressure of the organic phase.

The preparation of the compound of formula VI (reaction d) is carried out by acylation of the thiol V, as the sodium salt, with reactive derivatives of the 2-thiophencarboxylic acid; as reactive derivatives, acidic chloride or the mixed anhydrides are meant and the reaction conditions are the standard ones.

The obtention of the compound of formula IV (reaction e) takes place by reacting the bromoderivative IIb with thioglycolic acid in anhydrous environment in a suitable solvent, for example N,N-dimethylformamide, in the presence of a base as acceptor of hydrogen bromide, for example, triethylamine. The reaction takes place quickly +20°/+25°C and requires for the completion 4 hours.

The isolation of the compound IV takes place by diluting the reaction mass with water and subsequently acidificating to pH 2, whereby a crystalline product is obtained, which is filtered and then recrystallized.

The 3-mercapto-2-oxo-1-pyrrolidinyl acetic acid (V) and its derivatives are endowed with interesting pharmacological properties; particularly they are endowed with a relevant mucolytic expectorant and anti-cough activity and moreover show a very low toxicity. More specifically in the test of mucolytic activity "in vitro", with a 4% mucin suspension (Il Farmaco, ed. prat. 1978, 33, 379—391) and the molar concentration being the same (O, [125] M), the 3-mercapto-2-oxo-1-pyrrolidinyl acetic acid (V), the 3-(2-thenoylthio)-2-oxo-1-pyrrolidinyl acetic acid (VI) and 3-benzoylthio-2-oxo-1-pyrrolidinyl acetic acid (VII), (the last two ones after a suitable hydrolysis), caused a viscosity reduction respectively of 20.95%, 18.22% and 16.13%, which are significantly higher than that of N-acetyl-L-cysteine under the same experimental conditions (12.69%).

Furthermore the compounds of the present invention show a relevant expectorant activity, which is demonstrated in the test of fluorescein elimination in the fluid of the respiratory tract (Il Farmaco, ed. prat. 1981, 36, 167—172); in this test, two representative compounds, 3-mercapto-2-oxo-1-pyrrolidinyl acetic acid (V) and 3-(2-thenoylthio)-2-oxo-1-pyrrolidinyl acetic acid (VI) demonstrated, in comparison to the controls, increases of escretion of the colouring matter respectively of 52.92% and 54.26%, which are significantly higher than that of N-acetyl-L-cysteine at the same dose (1 mM/kg/os) and under the same experimental conditions (29.63%).

The anti-cough activity of the compounds of the present invention was assessed in the test of the cough induced by citric acid in the guinea pig (Screening methods in pharmacology of R. A. Turner -Acad. Press 1965, 219) wherein two representative compounds, 3-mercapto-2-oxo-1-pyrrolidinyl acetic acid (V) and 3-(2-thenoylthio)-2-oxo-1-pyrrolidinyl acetic acid (VI) did show with respect to the controls a reduction of the cough respectively equal to 74% and 68.50% at the dose of 1 mM/kg/i.p. which are not significantly different from that obtained with codeine phosphate at the dose of 5 mg/kg.i.p. (87.00%).

The compounds of the present invention have a very low acute toxicity; two representative compounds, 3-mercapto-2-oxo-1-pyrrolidinyl acetic acid (V) and 3-(2-thenoylthio)-2-oxo-1-pyrrolidinyl acetic acid (VI) show in the male and the female mouse a $LD_{50}$ value by i.p. route higher than 2000 mg/kg and a $LD_{50}$ value by oral route higher than 3000 mg/kg and in the male and female rat a $LD_{50}$ value by i.p. route higher than 1500 mg/kg and a $LD_{50}$ value by oral route higher than 5000 mg/kg. Thanks to their pharmacological properties and to the absence of toxicity, the compounds of formula I and their possible pharmaceutically acceptable salts may be used as drugs in the treatment of diseases of the respiratory tract.

Thus, the present invention relates, according to a further feature, to pharmaceutical compositions containing, as the active ingredients, the compounds of formula I or their possible pharmaceutically acceptable salts.

The pharmaceutical compositions of the present invention contain the active ingredients, both alone and in admixture with pharmaceutical vehicles or excipients, in pharmaceutical forms and in dosing units suitable for the administration by oral, parenteral, rectal or in-halating route, such as tablets, powders, granules, capsules, solutions, suspensions, syrups or suppositories.

Each dosage unit contains from 50 to 500 mg of active ingredient and is administered so as to give to the patient a daily dose of active substance varying from 100 to 2000 mg in the treatment of diseases of the respiratory organs, particularly bronchities, tracheobronchities, pharyngitis, rhinopharyngitis.

The compositions of the present invention may contain, besides the compounds of formula I or their possible salts, other active substances, such as for instance antibiotics, bronchodilating compounds, balsamic compounds, anti-inflammatory compounds, anti-hystaminic compounds or local anaesthetic compounds.

The following examples illustrate the invention.

## Example 1

A solution of 180 g (0.72 moles) of ethyl(3-bromo-2-oxo-pyrrolidin-1-yl)-acetate in 450 ml of 95% ethyl alcohol is added, at +15/+20°C and over 20 mintues, with 810 ml of a N solution of sodium hydroxide. The mixture is stirred for 4 hours at +15°C/+20°C, then neutralized with the equivalent amount of 3 N hydrogen chloride. The solution is evaporated under reduced pressure at +35°C until a dense syrupy mass is obtained, which is dispersed with 900 ml of ethyl acetate.

The mixture is made acidic to pH 3.5 with 6N hydrogen chloride, the temperature being maintained at +15°C/+20°C.

The organic phase is separated, and is collected, whereas the aqueous one is extracted after saturation with sodium chloride by means of further 600 ml of ethyl acetate. The combined ethly acetate phases are

dried on anhydrous sodium sulphate, decoloured with charcoal and stirred for 30 minutes. After filtration, the solution is evaporated under vacuum at +35°C until a white solid residue is obtained.

The raw product (110 g) is crystallized with 380 ml of anhydrous ethyl alcohol.

There are obtained: 98.5 g (55%) of 3-bromo-2-oxo-pyrrolidin-1-yl acetic acid; m.p. 155°—158°C.

Analitical data for $C_6H_8BrNO_2$ (MW 250.104)

Calculated %: C 28.81 H 3.22 N 5.60 Br 31.95

Found %: C 28.77 H 3.19 N 5.62 Br 31.79

The IR (KBr) and NMR (DMSO-$d_6$) spectra agree with the foreseen structure.

This compound is used as intermediate for preparing the compounds according to the invention.

Example 2

A solution of 29.4 g (0.35 moles) of sodium bicarbonate in 215 ml of water is added with 77.7 g (0.35 moles) of 3-bromo-2-oxo-pyrrolidin-1-yl acetic acid; separately a solution of potassium thiobenzoate is prepared from 53.6 g (0.388 moles) of thiobenzoic acid, 33.78 g (0.244 moles) of potassium carbonate and 357 ml of water. The latter solution is added to the former one under nitrogen stream over 30 minutes, and then the mixture is stirred for 5 hours at +20°C/+25°C (pH about 8).

Then 800 ml of ethyl acetate are added and the mixture is brought to pH 2 with 6 N hydrogen chloride; the organic phase is separated and the aqueous one is extracted with further 400 ml of ethyl acetate.

The combined organic extracts are dried over anhydrous sodium sulphate; after removal of the drying compound by filtration, and solid residue is obtained by evaporation under reduced pressure.

The raw product is crystallized with 370 ml ethyl acetate to give:

76 g (77%) of 3-benzoylthio-2-oxo-1-pyrrolidinyl acetic acid; m.p. 120°—122°C.

Analitical data for $C_{13}H_{13}NO_4S$ (MW 279.318)

Calculated %: C 55.90 H 4.69 N 5.015 S 11.48

Found %: C 56.03 H 4.66 N 4.99 S 11.50

A solution of 70 g (0.25 moles) of the thus obtained product in 110 ml of ethyl acetate, heated to 60°/65°C, is added with 250 ml of a solution of 1 M sodium 2-ethylhexanoate in ethyl acetate. After 30 minutes of stirring it is cooled to +5°C, filtered and washed with ethyl acetate (100 ml).

There are obtained: 65 g (86%) of crystalline white sodium 3-benzoylthio-2-oxo-pyrrolidin-1-yl acetate; m.p. 195—198°C.

Example 3

50 g (0.166 moles) of sodium 3-benzoylthio-2-oxo-pyrrolidin-1-yl acetate are suspended in 220 ml of water at +15°C/+20°C; under stirring there are added over 15 minutes 115 ml of a water solution of ammonium hydroxide 28° Be and a clear solution is obtained. It is maintained under stirring for 5 hours at +15°/+20°C, then it is cooled at +5°C and the crystalline product is filtered, it being formed by benzamide (16.2 g — 80% of the theoretical value). The filtrate is evaporated under vacuum at 50°C until a dense syrupy mass is obtained which is diluted with 80 ml of water and extracted with 200 + 100 ml of ethyl acetate to remove the benzamide still dissolved.

The aqueous phase is added with 300 ml of ethyl acetate and brought to pH 3 by addition of 37% hydrogen chloride; the organic phase is separated and the aqueous one is extracted with 200 + 100 ml of ethyl acetate.

The combined organic phases are dried over anhydrous sodium sulphate and evaporated under vacuum.

There are thus obtained: 20.7 g (71%) of 3-mercapto-2-oxo-pyrrolidin-1-yl acetate acid as a clear oil which solidificates completely: m.p. 78—81°C.

Analitical data for $C_6H_9NO_3S$ (MW 175.209)

Calculated %: C 41.13 H 5.17 N 7.99 S 18.30

Found %: C 41.21 H 5.12 N 7.98 S 18.34

The IR (KBr) and NMR ($CDCl_3$) spectra agree with the foreseen structure.

To a solution of 20 g (0.114 moles) of 3-mercapto-2-oxo-1-pyrrolidinyl acetic acid in 100 ml of ethyl acetate, heated to 50°C, are added 115 ml of a solution of 1 M sodium 2-ethylhexanoate in ethyl acetate. After 15 hours at +5°C, it is filtered and washed with ethyl acetate (30 ml).

There are thus obtained: 17.5 g (78%) of white crystalline sodium 1-carboxymethyl-2-oxo-3-pyrrolidinyl thiolate; m.p. 210—215°C.

Example 4

A solution of 50 g (0.25 moles) of the sodium salt obtained in the example 3 in 260 ml of water is added with 78.7 g (0.57 moles) of potassium carbonate.

It is cooled at +5°C, 36 g (0.31 moles) of chloride of 2-thiophencarboxylic acid are added, and the temperature is then allowed to raise to +15°C over 15 minutes. The reaction mass is poured in a mixture comprising 700 ml of methylene chloride and 350 ml of ethyl acetate, then brought to pH 2 by addition of 20% sulphuric acid, the temperature being maintained at +15°/+20°C.

The aqueous phase is separated and extracted with 500 ml of ethyl acetate/methylene chloride (1:2). The organic phases are dehydrated over anhydrous magnesium sulphate, concentrated under reduced pressure until a volume of 250 ml is obtained; after dilution with 300 ml of ethyl acetate, it is concentrated again under reduced pressure to 200 ml.

It is crystallized at +5°C for 24 hours, then filtered, washed with ethyl acetate (150 ml) and dried under reduced pressure at 35°C.

There are thus obtained: 45.8 g (64%) of 3-[2(-thienyl) carbonylthio]-2-oxo-pyrrolidin-1-yl acetic acid; m.p. 141—143°C.

Analitical data for $C_{11}H_{11}NO_4S_2$ (MW 285.344)

| | | | | |
|---|---|---|---|---|
| Calculated %: | C 46.30 | H 3.88 | N 4.90 | S 22.47 |
| Found %: | C 45.98 | H 3.88 | N 4.91 | S 22.44 |

The IR(KBr) and NMR(DMSO-$d_6$) spectra agree with the foreseen structure.

Example 5

To a solution of 7.5 g (0.03 moles) of 3-bromo-2-oxo-pyrrolidin-1-yl acetic acid in 100 ml of anhydrous, N,N-dimethylformamide there are subsequently added at +20°C/+25°C: 10.5 g (0.105 moles) of triethylamine and 4.15 g (0.045 moles) of thioglycolic acid.

The mixture is maintained under stirring for 4 hours at +20°C/+25°C, then the reaction mass is poured in 500 ml of cooled water (+5°C) and made acidic at pH 2 by addition of 37% hydrogen chloride.

The separation crystalline product is isolated by filtration, being then washed on the filter with cold water.

The raw product is crystallized from ethanol/water = 90:10 and there are obtained: 6.3 g (90%) of 3-carboxymethylthio-2-oxo-pyrrolidin-1-yl acetic acid; m.p. 54—55°C.

Analitical data for $C_8H_{11}NO_5S$ (MW 233.247)

| | | | | |
|---|---|---|---|---|
| Calculated %: | C 41.19 | H 4.75 | N 6.00 | S 13.75 |
| Found %: | C 41.12 | H 4.68 | N 5.96 | S 13.77 |

Example 6

A solution of 12.05 g (0.04 moles) of the sodium salt obtained in the example 3 in 60 ml of water is added with 8.7 g (0.082 moles) of sodium carbonate. The temperture is brought to +10°C and there are added 6.52 (0.05 moles) of the chloride of the 2-furancarboxylic acid, then, after 30 minutes of stirring at +10°/+15°C the reaction mass is diluted with 120 ml of methylene chloride. It is brought to pH 1.7 with 3N hydrogen chloride, the temperature being maintained at +15°/+20°C; the organic phase is separated, washed with 20 ml × 2 of water, then dehydrated, over anhydrous sodium sulphate and evaporated under reduced pressure until a volume of 50 ml is obtained.

The residual solution is diluted with 120 ml of ethyl ether and then crystallized at +5°C for 15 hours. It is filtered, washed with ethyl ether (50 ml) and dried under vacuum at 35°C. There are thus obtained: 8.8 g (82%) of 3-[(2-furyl) carbonylthio]-2-oxo-pyrrolidin-1-yl acetic acid; m.p. 158—160°C.

Analitical data for $C_{11}H_{11}NO_5S$ (MW 269.28)

| | | | | |
|---|---|---|---|---|
| Calculated %: | C 49.06 | H 4.12 | N 5.20 | S 11.91 |
| Found %: | C 48.88 | H 4.09 | N 5.20 | S 11.84 |

Example 7

21.2 g (0.2 moles) of sodium carbonate are dissolved in 200 ml of water and the solution is added with 100 g (0.4 moles) of 3-bromo-2-oxo-1-pyrrolidinacetic acid; thereafter there is added over 30 minutes under nitrogen a solution of 33.5 g (0.44 moles) of thioacetic acid, 31.3 g (0.295 moles) of sodium carbonate and 235 ml of water. After 4 hours of stirring at +20°C, 450 ml of ethyl acetate are added and the pH is brought to 2 by means of 6 N hydrogen chloride. The aqueous phase is separated and after addition of 150 g of sodium chloride is extracted with further 200 ml of ethyl acetate.

The combined phases of ethyl acetate are dehydrated with anhydrous sodium sulphate; after removal of the drying substance by filtration, it is evaporated under reduced pressure until a dense oil of yellow colour is obtained. The raw product is purified by distillation under vacuum. There are thus obtained: 48.6 g (56%) of 3-acetylthio-2-oxo-pyrrolidin-1-yl acetic acid; b.p. 180—185°C ($4.10^{-5}$ bar).

Analitical data for $C_8H_{11}NO_4S$ (MW 217.25)

| | | | | |
|---|---|---|---|---|
| Calculated %: | C 44.23 | H 5.10 | N 6.44 | S 14.75 |
| Found %: | C 44.85 | H 5.07 | N 6.47 | S 14.68 |

The IR (film) and NMR ($CDCl_3$) spectra are in agreement with the foreseen structure.

Example 8

A solution of 7.3 g (0.053 moles) of thiobenzoic acid and 8.8 g (0.053 moles) of sodium 2-ethylhexanoate in 30 ml of anhydrous ethyl alcohol is added to 16.5 g (0.66 moles) of ethyl 3-bromo-2-oxo-pyrrolidin-1-yl acetate dissolved in 50 ml of anhydrous ethyl alcohol, the temperature being maintained at +20°C/+25°C.

After 4 hours of stirring at +20°C/+25°C, the solution is evaporated under reduced pressure, then the residue is taken with 20 ml of water and 200 ml of ethyl acetate. The ethyl acetate phase is separated, washed with 50 ml × 2 of an aqueous solution of 2% sodium bicarbonate, then with 50 ml × 2 of water.

After drying over anhydrous sodium sulphate the organic phase is evaporated under reduced pressure.

The product is purified by distillation under vacuum to give: 11.3 g (69%) of ethyl 3-benzoylthio-2-oxo-pyrrolidin-1-yl acetate, in form of a yellow oil; b.p. 185°—188°C ($9.3.10^{-6}$ bar).

Analitical data for $C_{15}H_{17}NO_4S$ (MW 307.372)

| | | | | |
|---|---|---|---|---|
| Calculated %: | C 58.61 | H 5.57 | N 4.56 | S 10.43 |
| Found %: | C 57.98 | H 5.53 | N 4.55 | S 10.45 |

IR (film) and NMR ($CDCl_3$) spectra are in agreement with the foreseen structure.

Example 9

A solution of 6.025 g (0.02 moles) of the sodium salt described in the example 3 in 30 ml of water, is added with 4.35 g (0.041 moles) of sodium carbonate.

There is subsequently added at +5°/+10°C a solution of 3.7 g (0.025 moles) of the chloride of the 1,3,4-thiadiazol-2-yl-carboxylic acid in 15 ml of ethyl acetate; after 1 hour of stirring at the same temperature, the pH of the mixture is brought to 1.8 with 2N hydrogen chloride. The organic phase is separated, washed with 20 ml × 2 of water, dehydrated over anhydrous sodium sulphate and evaporated at reduced pressure until a solid residue is obtained. The residue is taken with 65 ml of ethyl ether, filtered, washed with ethyl ether (30 ml) and dried under vacuum at 35°C.

There are thus obtained: 4.8 g (83.6%) of 3-[(1,3,4-thiadiazol-2-yl)carbonylthio]-2-oxo-pyrrolidin-1-yl acetic acid; m.p. 172—174°C (dec.)

Analitical data for $C_9H_9N_3O_4S_2$ (MW 287.32)

| | | | | |
|---|---|---|---|---|
| Calculated %: | C 37.62 | H 3.16 | N 14.62 | S 22.32 |
| Found %: | C 37.81 | H 3.11 | N 14.44 | S 21.98 |

Example 10

Vials based on sodium 3-[(2-thienyl) carbonylthio]-2-oxo-pyrrolidin-1-yl acetate are prepared, having the following composition:

| | |
|---|---|
| 3-[(2-thienyl) carbonylthio]-2-oxo-pyrrolidin-1-yl acetic acid | 100 mg |
| sodium carbonate | 37 mg |
| apirogenic distilled water q.s. to | 3 ml |

In the same manner vials containing 200 and 300 mg of active principle are prepared.

Example 11

A syrup is prepared based on one of the compounds of the examples 2 to 9, having the following composition:

| | |
|---|---|
| active ingredient | 2 g |
| 70% sorbitol | 15 g |
| saccharose | 50 g |
| ethyl alcohol | 1 ml |
| sodium p-hydroxybenzonate | 0.2 mg |
| flavouring essences | 0.5 ml |
| saccharin | 0.2 g |
| distilled water q.s. to | 100 ml |

In the same manner a syrup containing 3 g of active principle in 100 ml of syrup is prepared, corresponding to the unit dose of 300 mg.

Example 12

Suppositories are prepared based on one of the compounds of examples 2 to 9, having the following composition:

| | |
|---|---|
| active ingredient | 200 mg |
| triglycerides of saturated fatty acids | 2800 mg |

The finely powdered active substance is poured, under stirring, in the melted mass for suppositories, at the temperature of 40°C.

The mass is then placed into the moulds which have been previously slightly cooled.

Weight of each suppository: 3 g

Likewise suppositories of 3 g containing 300 mg of active principle, suppositories of 2 g containing 100 mg of active principle and suppositories of 2 g containing 150 mg of active principle are prepared.

Example 13

A granulate is prepared for the recoustitution of an oral liquid formulation based on one of the compounds of the examples 2 to 9, having the following composition:

| | |
|---|---|
| active principle | 4 g |
| saccharine | 0.16 g |
| orange flavour | 0.40 g |
| orange lyophilized extract | 10 g |
| saccharose q.s. to | 100 g |

The granulate, prepared according to the standard methods, is filled into small envelopes containing 5 g of the same granulate.

The content of one small envelope (200 mg of active principle), dissolved into the water, constitutes a suitable unit dose to be administered to adult patients one to three times per day during the treatment, for instance of bronchitis.

In the same manner small envelopes are prepared containing 5 g of granulate, wherein 300 mg of active principle are present.

Example 14

By operating as described in the example 13, small envelopes are prepared containing 5 g of granulate having the following composition:

| | |
|---|---|
| active principle | 2 g |
| saccharine | 0.16 g |
| orange flavour | 0.40 g |
| orange lyophilized extract | 10 g |
| saccharose q.s. to | 100 g |

Each small envelope thus prepared contains 100 mg of active principle.

Likewise small envelopes containing 150 mg of active principle are prepared.

Example 15

Capsules based on one of the compounds of the examples 2 to 9 are prepared, having the following composition:

| | |
|---|---|
| active principle | 200 mg |
| powdered lactose | 100 mg |

The ingredients are thoroughly admixed so that the active principle is distributed in the lactose mass.

The powder is then poured into empty gelatine capsules of the size 1.

Likewise capsules containing 300 mf active principle are prepared.

**Claims**

1. A compound having the formula

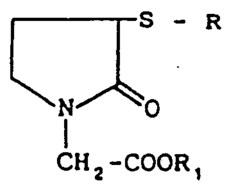

(I),

wherein R is hydrogen, $—COR_2$, $—CH_2COOR_3$, in which $R_2$ is a $C_1—C_8$ linear or branched chain alkyl, or a phenyl, or a heterocyclic radical containing 1, 2 or 3 hetero atoms selected from among sulphur, nitrogen, oxygen, and in which $R_3$ is hydrogen, a linear or branched chain $C_1—C_4$ alkyl, and $R_1$ is hydrogen or a linear or branched chain $C_1—C_8$ alkyl.

2. A compound according to claim 1 wherein R is a group $—COR_2$ in which $R_2$ is 2-thienyl, 2-furyl, 4-thiazolyl, 1,3,4-thiadiazol-2-yl.

3. Pharmaceutically acceptable salt of a compound according to claim 1 in which one or both of the substituents R and $R_1$ or of the substituents $R_3$ and $R_1$, are hydrogen.

4. A process for the preparation of the compound of the claim 1, characterized by reacting a 3-bromo-pyrrolidin-2-one derivative of the formula II

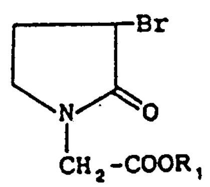

(II),

wherein $R_1$ is as defined in the claim 1, with a thiol of the formula III

HS—R (III)

wherein R is defined as in claim 1, or with a alkaline salt thereof, optionally in the presence of inorganic or organic bases, in an organic solvent at 0°C to 40°C, and optionally the resultant product is converted into its pharmaceutically acceptable salt.

5. Modification of the process in accordance with claim 4, characterized by reacting the 3-bromo-pyrrolidin-2-one derivative of the formula II, wherein $R_1$ is hydrogen, with a alkaline salt of the thiol of formula III, wherein R is benzoyl, then subjecting to hydrolysis the thus obtained product and the 3-mercapto-2-oxo-pyrrolidin-1-yl acetic acid is isolated.

6. Process in accordance with the modification of the claim 5 characterized by the reaction of the 3-mercapto-2-oxo-pyrrolidin-1-yl acetic acid with a reactive derivative of the 2-thiophencarboxylic acid and by the optional conversion of the 3-[(2-thienyl) carbonylthio]-2-oxo-pyrrolidin-1-yl acetic acid into a pharmaceutically acceptable salt thereof.

7. A pharmaceutical composition containing as the active ingredient a compound as defined in any one of the claims 1 to 3.

8. A pharmaceutical composition according to claim 7, characterized by being formulated in dosage units.

9. A pharmaceutical composition according to claim 8, containing from 100 to 500 mg of the active ingredient for dosage unit in admixture with a pharmaceutical excipients.

**Patentansprüche**

1. Verbindung der Formel

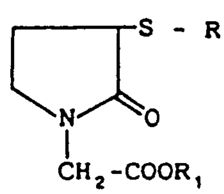

(I),

worin R Wasserstoff, $—COR_2$ oder $—CH_2COOR_3$ bedeutet, wobei $R_2$ gerad- oder verzweigtkettiges $C_1—C_8$-Alkyl, Phenyl oder ein heterocyclischer Rest mit 1, 2 oder 3 Heteroatomen ausgewählt aus Schwefel, Stickstoff und Sauerstoff ist und $R_3$ Wasserstoff oder gerad- oder verzweigtkettiges $C_1—C_4$-Alkyl bedeutet, und $R_1$ Wasserstoff oder gerad- oder verzweigtkettiges $C_1—C_8$-Alkyl darstellt.

2. Verbinding nach Anspruch 1, worin R eine Gruppe —$COR_2$ ist, wobei $R_2$ 2-Thienyl, 2-Furyl, 4-Thiazolyl oder 1,3,4-Thiadiazol-2-yl bedeutet.

3. Pharmazeutisch annehmbares Salz einer Verbindung nach Anspruch 1, in dem einer oder beide der Substituenten R und $R_1$ oder der Substituenten $R_3$ und $R_1$ Wasserstoff sind.

4. Verfahren zur Herstellung der Verbindung nach Anspruch 1, dadurch gekennzeichnet, daß ein Brompyrrolidin-2-on-Derivat der Formel (II)

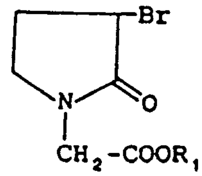

(II),

worin $R_1$ wie in Anspruch 1 definiert ist, mit einem Thiol der Formel (III)

$$HS—R \quad (III),$$

worin R wie in Anspruch 1 definiert ist, oder mit einem Alkalisalz hievon, gegebenenfalls in Anwesenheit von anorganischen oder organischen Basen, in einem organischen Lösungsmittel bei 0 bis 40°C umgesetzt und das erhaltene Produkt gegebenenfalls in ein pharmazeutisch annehmbares Salz hievon übergeführt wird.

5. Abänderung des Verfahrens nach Anspruch 4, dadurch gekennzeichnet, daß das 3-Brompyrrolidin-2-on-Derivat der Formel (II), worin $R_1$ Wasserstoff ist, mit einem Alkalisalz des Thiols der Formel (III), worin R Benzoyl darstellt, umgesetzt, dann das so erhaltene Produkt hydrolysiert und die 3-Mercapto-2-oxo-pyrrolidin-1-yl-essigsäure isoliert wird.

6. Verfahren gemäß der Abänderung von Anspruch 5, dadurch gekennzeichnet, daß die 3-Mercapto-2-oxo-pyrrolidin-1-yl-essigsäure mit einem reaktiven Derivat der 2-Thiophencarbonsäure umgesetzt und die 3-[(2-Thienyl)-carbonylthio]-2-oxo-pyrrolidin-1-yl-essigsäure gegebenenfalls in ein pharmazeutisch annehmbares Salz hievon übergeführt wird.

7. Pharmazeutische Zusammensetzung, die als Wirkstoff eine Verbindung, wie in einem der Ansprüche 1 bis 3 definiert, enthält.

8. Pharmazeutische Zusammensetzung nach Anspruch 7, dadurch gekennzeichnet, daß sie in Dosierungseinheiten formuliert ist.

9. Pharmazeutische Zusammensetzung nach Anspruch 8, die 100 bis 500 mg Wirkstoff pro Dosierungseinheit in Mischung mit einem pharmazeutischen Exzipienten enthält.

**Revendications**

1. Composé de formule

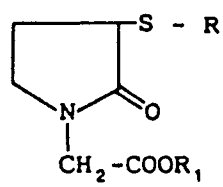

(I),

dans laquelle R est l'hydrogène, —$COR_2$, —$CH_2COOR_3$, où $R_2$ est une chaîne alkyle linéaire ou ramifié, ou un radical phényl ou hétérocyclique contenant, 1, 2 ou 3 hétéro-atomes choisis parmi le soufre, l'azote et l'oxygène, et où $R_3$ est l'hydrogène, une chaîne alkyle en $C_1$ à $C_4$ linéaire ou ramifié et $R_1$ est l'hydrogène ou une chaîne alkyle en $C_1$ à $C_8$ linéaire ou ramifiée.

2. Composé selon la revendication 1, dans lequel R est un groupe —$COR_2$, où $R_2$ est le 2-thiényle, le 2-furyle, le 4-thiazolyle, le 1,3,4-thiadiazol-2-yle.

3. Sel, acceptable dans la domaine pharmaceutique, d'un composé selon la revendication 1, dans lequel l'un ou les deux des substituants R et $R_1$ ou des substituants $R_3$ et $R_1$ sont l'hydrogène.

4. Procédé de préparation du composé selon la revendication 1, caractérisé par le fait que l'on fait réagir un dérivé 3-bromo-pyrrolidin-2-one de formule (II)

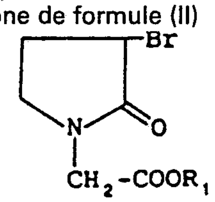

(II),

dans lequel $R_1$ est tel que défini dans la revendication 1, avec un thiol de formule (III)

$$HS—R \qquad (III)$$

dans lequel R est tel que défini dans la revendication 1, ou avec un de ses sels alcalins, facultativement en présence de bases minérales ou organiques, dans une solvant organique entre 0°C et 40°C et le produit résultant étant facultativement converti en son sel acceptable dans le domaine pharmaceutique.

5. Modification du procédé selon la revendication 4, caractérisée par le fait que l'on fait réagir le dérivé 3-bromo-pyrrolidin-2-one de formule (II), dans lequel $R_1$ est l'hydrogène, avec un sel alcalin du thiol de la formule (III) dans laquelle R est le radical benzoyle, puis en soumettant à l'hydrolyse le produit ainsi obtenu et ensuite en isolant l'acide 3-mercapto-2-oxo-pyrrolidin-1-yle acétique.

6. Procédé selon la modification indiquée dans la revendication 5, caractérisé par le fait que l'on fait réagir l'acide 3-mercapto-2-oxo-pyrrolidin-1-yle acétique avec un réactif dérivé de l'acide 2-thiophène carboxylique et par la conversion facultative de l'acide 3-[(2-thiényl)carbonylthio]-2-oxo-pyrrolidin-1-yle acétique en un sel de celui-ci acceptable dans le domaine pharmaceutique.

7. Composition pharmaceutique contenant en tant que corps actif un composé tel que défini dans l'une quelconque des revendications 1 à 3.

8. Composition pharmaceutique selon la revendication 7, caractérisée par le fait qu'elle est mise sous forme de doses unitaires.

9. Composition pharmaceutique selon la revendication 8, contenant 100 à 500 mg du corps actif par dose unitaire, incorporée avec un excipient pharmaceutique.